Europäisches Patentamt

**European Patent Office**

**Office européen des brevets**

(11) Publication number: **0 096 557**
**B1**

(12) ## EUROPEAN PATENT SPECIFICATION

(45) Date of publication of patent specification: **05.11.86**

(21) Application number: **83303222.0**

(22) Date of filing: **03.06.83**

(51) Int. Cl.⁴: **G 01 N 27/56,** G 01 N 33/00 //
G01N1/24, G01N33/22

(54) **Determining the concentration of oxygen and combustibles within a gas.**

(30) Priority: **04.06.82 US 385201**

(43) Date of publication of application:
**21.12.83 Bulletin 83/51**

(45) Publication of the grant of the patent:
**05.11.86 Bulletin 86/45**

(84) Designated Contracting States:
**DE FR GB IT**

(56) References cited:
**WO-A-82/03689**
**US-A-4 128 458**
**US-A-4 134 289**

(73) Proprietor: **THE BABCOCK & WILCOX COMPANY**
**1010 Common Street P.O. Box 60035**
**New Orleans Louisiana 70160 (US)**

(72) Inventor: **Barnett, Daniel C.**
**10100 Chipmunk Ridge**
**Concord Twonship Ohio 44077 (US)**
Inventor: **Zimmerlin, Sharon L.**
**180 Elm Ct.**
**Chagrin Falls Ohio 44022 (US)**

(74) Representative: **Cotter, Ivan John et al**
**D. YOUNG & CO. 10 Staple Inn**
**London WC1V 7RD (GB)**

Courier Press, Leamington Spa, England.

## Description

This invention relates to devices for determining the concentration of oxygen and combustibles within a gas.

Apparatus has been available for determining the combustibles and oxygen concentration in a combustible fuel environment. By measuring these parameters and taking appropriate corrective action in response thereto, it is possible to maintain the proper environment for the most efficient burning of the combustible.

As an example, during the combustion of carbon monoxide in an oxygen atmosphere, the most efficient burning of the carbon monoxide occurs when two moles of carbon monoxide are present for each mole of oxygen. If the actual carbon monoxide/oxygen ratio is greater than or less than this ratio, inefficient combustion results. If the carbon monoxide concentration is too high, the excess carbon monoxide tends to inhibit the combustion process and the burning becomes extinguished. In contrast, if the carbon monoxide concentration is too low, there is an excess of oxygen and the system becomes inefficient inasmuch as it must heat the excess oxygen. Thus, it is desirable and advantageous to monitor the carbon monoxide and the oxygen concentrations during the combustion process and to regulate same so as to ensure complete combustion.

The prior art discloses various systems for monitoring the relative concentrations of both oxygen and combustibles in a burning environment. However, these systems typically require a separate aspirator for each gas constituent analysed, as disclosed in US Patent No. 4 134 289. This approach requires considerable "hardware" to implement the system and flow optimisation is difficult to achieve. Some systems such as that of US Patent No. 4 128 458 use a single aspirator to achieve flow therethrough. However, it has been found that these systems do not provide for effective proportioning of the flow between the oxygen analyser and the combustibles analyser. Thus, flow proportioning does not exist in these systems. Moreover, in the situation where dilution air is supplied to the combustibles analyser, backflow of this dilution air may occur to the oxygen analyser thereby causing the oxygen reading to be inaccurate.

Because of the foregoing, it has become desirable to develop an improved device or system for monitoring both the oxygen and the combustibles concentration in a burning environment, more particularly (but not exclusively) such a device or system utilizing only one aspirator and achieving flow proportioning between an oxygen analyser and a combustibles analyser.

According to the invention there is provided a device for determining the concentration of oxygen and combustibles within a gas, the device comprising an oxygen concentration analyser, a combustibles concentration analyser, conduit means including first and second conduits for respectively conveying a flow of gas through the oxygen concentration analyser and the combustibles concentration analyser, an inlet to the conduit means for receiving a sample of gas to be analysed, an outlet from the conduit means for discharging said sample after completion of analysis thereof, means for producing a flow of gas in the conduit means, and means for proportioning the flow of the sample between the first and second conduits, characterised in that the proportioning means comprises means for allowing a higher volume of sample to flow to the oxygen concentration analyser than to the combustibles concentration analyser and for preventing backflow between the conduits.

A preferred embodiment of the present invention described hereinbelow solves the aforementioned problems associated with the prior art by providing a device or system which utilizes a single aspirator for maintaining the flow of the gas to both an oxygen analyser and a combustibles analyser. The aspirator is driven by a source of compressed air. A plurality of orifices is provided to regulate the pressure to the aspirator, to drop the sample pressure to a pre-determined level, to proportion the flows to the oxygen analyser and to the combustibles analyser, and to prevent backflow between the oxygen and the combustibles analyser.

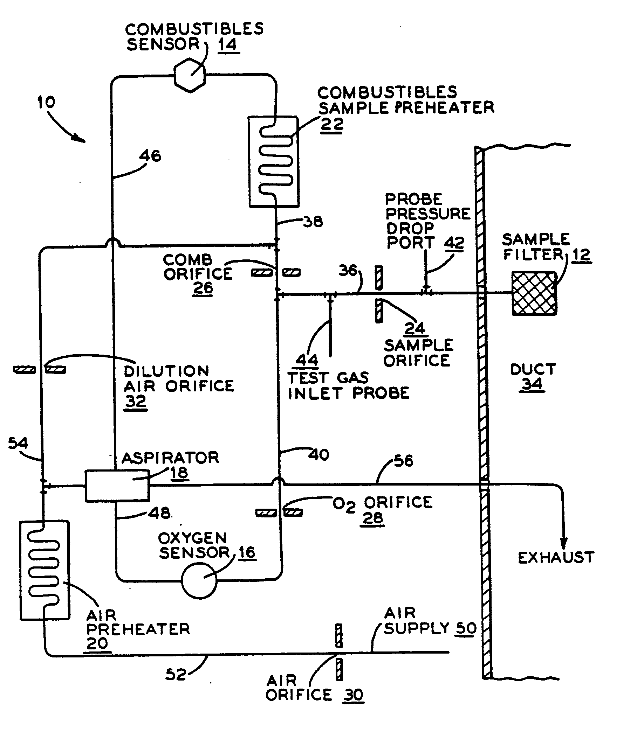

The invention will now be further described, by way of illustrative and non-limiting example, with reference to the accompanying drawing, the sole figure of which is a schematic representation of a device or system embodying the invention.

The sole figure of the accompanying drawing is a schematic representation or a device or system 10 embodying the present invention. The system 10 comprises a sample filter 12, a combustibles sensor 14, an oxygen sensor 16, an aspirator 18, an air preheater 20, a combustibles sample preheater 22, and a plurality of orifices comprising a sample orifice 24, a combustibles orifice 26, an oxygen orifice 28, an air orifice 30 and a dilution air orifice 32.

The sample filter 12, which is of standard design, is inserted into a duct 34 carrying a gas to be analysed. An output of the sample filter 12 is connected via a conduit 36, which is subsequently divided into two conduits 38 and 40, to inputs to the combustibles sensor 14 and the oxygen sensor 16, respectively. A probe pressure drop port 42, the sample orifice 24, and a test gas inlet port 44 are provided in the conduit 36. The combustibles sample preheater 22 and the combustibles orifice 26 are provided in the conduit 38 prior to connection thereof to the input to the combustibles sensor 14, whereas the oxygen orifice 28 is provided in the conduit 40 prior to its connection to the input to the oxygen sensor 16. Outputs of the combustibles sensor 14 and the oxygen sensor 16 are connected to inputs to the aspirator 18 via conduits 46 and 48, respectively. An air supply 50, at a pressure of approximately 69 kPa (10lbf/in$^2$ gauge), is connected to another input to the aspirator 18 via a conduit 52. The air orifice 30 and the air preheater 20 are provided in the conduit 52 prior to its connection to an input to the aspirator 18. The air supply 50 is also connected to the input to the combustibles sample preheater 22 via a conduit 54 in which the dilution air orifice 32 is provided. An output of the aspirator 18 is connected via an exhaust conduit 56 to the duct 34.

In operation, fluid flow throughout the device or system 10 is maintained by the aspirator 18, which is powered by the air supply 50. The air orifice 30 regulates the air pressure to the aspirator 18 to 17 kPa (2.5 lbf/in$^2$ gauge), making the aspirator 18 virtually immune to changes in the pressure of the air supply 50. The aspirator 18 causes approximately 2000 cm$^3$/min of gas to be extracted from the duct 34 through the sample filter 12 and into the conduit 36 for analysis purposes. The pressure drop across the sample filter 12, as measured by the probe pressure drop port 42, is approximately $-250$ Pa ($-25$ mm H$_2$ or $-1$ inch H$_2$O) when the pressure within the duct 34 is OPa (0 mm H$_2$O). As the gas sample passes through the sample orifice 24 its pressure decreases to $-1.5$ kPA ($-152$ mm H$_2$O or $-6$ inches H$_2$O) so as to provide less sensitivity to filter plugging. The gas sample flow then divides between the conduits 38 and 40 and is regulated by the orifices 26 and 28, respectively, so that approximately 800 cm$^3$/min of gas flows to the combustible sensor 14 and approximately 1200 cm$^3$/min of gas flows to the oxygen sensor 16. The dilution air orifice 32 allows approximately 200 cm$^3$/min of air, via the conduit 54, to be mixed with the 800 cm$^3$/min of gas flowing to the combustibles sensor 14, via the conduit 38. This addition of air to the flow of gas to the combustibles sensor 14 ensures combustion thereof at the combustibles sensor 14. The combustion orifice 26 regulates the flow of gas to the combustibles sensor 14 and also prevents the diluted combustibles sample flow from backflowing, via the conduit 40, to the oxygen sensor 16.

The combustibles sensor 14 is typically of the thermopile type and thus operates at an elevated temperature. Because of this, the combustibles sample preheater 22 heats the sample to approximately 316°C (600°F) to ensure its combustion at the combustibles sensor 14. The oxygen sensor 16 is typically of the zirconium oxide type and is similar to that disclosed in US Patent No. 3 960 500. The gas sample which passes through the sensor 16 interacts with the sensor to produce an electrical output indicative of the oxygen concentration. After the samples have been analysed by the combustibles sensor 14 and the oxygen sensor 16, the diluted combustibles sample (approximately 1000 cm$^3$/min) and the oxygen sample flow (approximately 1200 cm$^3$/min) flow via their respective conduits 46 and 48 to the aspirator 18, in which they are combined and discharged to the duct 34 via the conduit 56. The location of the exhaust exit into the duct 34 is sufficiently downstream from the sample filter 12 as to ensure that the incoming gas to the sample filter 12 is not contaminated by the gas already analysed.

From the foregoing, it is apparent that only one aspirator, rather than two aspirators, as in the prior art, is required for the analysis of both the combustibles and the oxygen concentrations of the gas, provided that suitable orifices are utilized for proportioning the gas between the analysers. Thus, the amount of "hardware" required for gas analysis is reduced and flow optimisation is achieved by this system.

**Claims**

1. A device for determining the concentration of oxygen and combustibles within a gas, the device (10) comprising an oxygen concentration analyser (16), a combustibles concentration analyser (14), conduit means including first and second conduits (40, 38) for respectively conveying a flow of gas through the oxygen concentration analyser (16) and the combustibles concentration analyser (14), an inlet to the conduit means for receiving a sample of gas to be analysed, an outlet from the conduit means for discharging said sample after completion of analysis thereof, means (18) for producing a flow of gas in the conduit means, and means for proportioning the flow of the sample between the first and second conduits, characterised in that the proportioning means comprises means (26, 28) for allowing a higher volume of sample to flow to the oxygen concentration analyser (16) than to the combustibles concentration analyser (14) and for preventing backflow between the conduits (40, 38).

2. A device according to claim 1, wherein the means for producing a flow comprises a single aspirator (18) operative to produce pressure variations within the conduit means, and wherein the conduit means includes an air supply conduit (52) for directing a supply (50) of air to the aspirator (18) to power the aspirator.

3. A device according to claim 2, wherein an air orifice (30) is provided within the air supply conduit (52) between the aspirator (18) and the air supply (50) to make the aspirator (18) insensitive to variations in the air supply (50).

4. A device according to claim 3, wherein the air supply conduit (52) includes means (20) for heating the air supply used to power the aspirator (18).

5. A device according to claim 3 or claim 4, wherein the air supply conduit (52) is arranged also to direct the supply of air to the combustibles concentration analyser (14) to dilute the flow of the sample to the combustibles concentration analyser.

6. A device according to claim 5, including means (22) for heating the diluted flow of the sample to the combustibles concentration analyser (14).

7. A device according to claim 5 or 6, including a further air orifice (32) downstream of the first-mentioned air orifice (30) and sized so as to supply about 200 cm$^3$/min of dilution air to the combustibles concentration analyser (14).

8. A device according to any one of the preceding claims, wherein the proportioning means comprises a plurality of orifices (24, 26, 28) positioned within conduits (36, 38, 40) of the conduit means to proportion the flow of the sample between the oxygen concentration analyser (16) and the combustibles concentration analyser (14).

9. A device according to any one of the preceding claims, wherein the proportioning means is operable to proportion the flow of the sample between the oxygen concentration analyser (16) and the combustibles concentration analyser (14) according to a ratio approximately 3 to 2.

10. A device according to claim 9, wherein the sample flow to the oxygen concentration analyser (16) is about 1200 cm³/min and to the combustibles concentration anaylser (14) is about 800 cm³/min.

**Patentansprüche**

1. Vorrichtung zur Bestimmung der Konzentration von Sauerstoff und Brennstoffen in einem Gas, wobei die Vorrichtung (10) einen Sauerstoffkonzentrations-Analysator (16), einen Brennstoffkonzentration-Analysator (14), Leitungen einschließlich einer ersten und einer zwieten Leitung (40, 38,) für jeweilige Beförderung eines Gasstromes durch den Sauerstoffkonzentrations-Analysator (16) bzw. den Brennstoffkonzentrations-Analysator (14), einen Einlaß zu den Leitungen zur Aufnahme einer Probe des zu analysierenden Gases, einen Auslaß den Leitungen zur Entnahme dieser Probe nach vollständiger Analyse derselben, Einrichtungen (18) zur Erzeugung eines Gasstromes in den Leitungen und Einrichtungen zur Dosierung des Stromes der Probe zwischen der ersten und zweiten Leitung aufweist, dadurch gekennzeichnet, daß die Dosiereinrichtungen Einrichtungen (26, 28) aufweisen, die ein größeres Probeuvolumen zu dem Sauerstoffkonzentrations-Analysator (16) als zu dem Brennstoffkonzentrations-Analysator (14) erlauben und einen Rückfluß zwischen den Leitungen (40), verhindern.

2. Vorrichtung nach Anspruch 1, bei der die Einrichtung zur Erzeugung eines Stromes einen derart arbeitenden einzelnen Aspirator (18), daß er Druckänderungen in den Leitungen erzeugt, aufweist und bei der die Lietungen eine Luftzufuhrleitung (52) zur Führung einer Zufuhr (50) von Luft zu dem Aspirator (18), um den Aspirator zu betätigen, einschließen.

3. Vorrichtung nach Anspruch 2, bei der eine Luftöffnung (30) in der Luftzufuhrleitung (52) zwischen dem Aspirator (18) und der Luftzufuhr (50) vorgesehen ist, um den Aspirator (18) gegenüber Veränderungen in der Luftzufuhr (50) unempfindlich zu machen.

4. Vorrichtung nach Anspruch 3, bei der Luftzufuhrleitung (52) Einrichtungen (20) zum Erhitzen der Luftzufuhr, die zur Betätigung des Aspirators (18) verwendet wird, einschließt.

5. Vorrichtung nach Anspruch 3 oder Anspruch 4, bei der die Luftzufuhrleitung (52) auch so angeordnet ist, daß sie die Luftzufuhr zu dem Brennstoffkonzentrations-Analysator zu verdünnen.

6. Vorrichtung nach Anspruch 5 mit Einrichtungen (22) zum Erhitzen des verdünnten Stromes der Probe zu dem Brennstoffkonzentrations-Analysator (14).

7. Vorrichtung nach Anspruch 5 oder Anspruch 6 mit einer weiteren Luftöffnung (32) abstromwärts von der ersterwähnten Luftöffnung (30) und mit solcher Bemessung, daß sie etwa 200 cm³/min Verdünnungsluft zu dem Brennstoffkonzentration-Analysator (14) zuführt.

8. Vorrichtung nach einem der vorausgehenden Ansprüche, bei der die Dosiereinrichtung mehrere Öffnungen (24, 26, 28) aufweist, die in Leitungen (36, 38, 40) der Leitungseinrichtungen angeordnet sind, um den Strom der Probe zwischen dem Sauerstoffkonzentrations-Analysator (16) und dem Brennstoffkonzentrations-Analysator (14) zu dosieren.

9. Vorrichtung nach einem der vorausgehenden Ansprüche, bei der die Dosiereinrichtung so arbeitet, daß sie den Strom der Probe zwischen dem Sauerstoffkonzentrations-Analysator (16) und dem Brennstoffkonzentrations-Analysator (14) entsprechend einem Verhältnis von etwa 3:2 dosiert.

10. Vorrichtung nach Anspruch 9, bei der der Probenstrom zu dem Sauerstoffkonzentrations-Analysator (16) etwa 1200 cm³/min und zu dem Brennstoffkonzentrations-Analysator (14) etwa 800 cm³/min beträgt.

**Revendications**

1. Dispositif de détermination de la concentration en oxygène et en combustibles dans un gaz, le dispositif (10) comprenant un analyseur de concentration en oxygène (16), un analyseur de concentration en combustibles (14), des moyens de canalisation comportant un premier et un second conduits (40, 38) pour envoyer un courant de gaz traverser respectivement l'analyseur de concentration en oxygène (16) et l'analyseur de concentration en combustibles (14), une entrée desservant les moyens de canalisation pour recevoir un échantillon de gaz à analyseur, une sortie desservant les moyens de canalisation pour évacuer ledit échantillon après achèvement de son analyse, des moyens (18) pour l'établissement d'un courant de gaz dans les moyens de canalisation, et des moyens de répartition proportionnelle du courant d'échantillon entre les premier et second conduits, caractérise en ce que les moyens de répartition proportionnelle comprennent des moyens (26, 28) propres à assurer l'envoi d'un plus grand volume d'échantillon à l'analyseur de concentration en oxygène (16) qu'à l'analyseur de concentration en combustibles (14) et à empêcher le refluement entre les conduits (40, 38).

2. Dispositif selon la revendication 1, dans lequel les moyens d'établissement d'un courant comprennent un seul aspirateur (18) assurant l'apparition de variations de pression à l'intérieur des moyens de canalisation, et dans lequel les moyens de canalisation comportent un conduit d'alimentation d'air (52) pour diriger une alimentation (50) d'air vers l'aspirateur (18) pour actionner celui-ci.

3. Dispositif selon la revendication 2, dans lequel un orifice à air (30) est prévu à l'intérieur du conduit d'alimentation d'air (52) entre l'aspirateur (18) et l'alimentation d'air (50) pour rendre l'aspirateur (18) insensible à des variations de l'alimentation d'air (50).

4. Dispositif selon la revendication 3, dans lequel le conduit d'alimentation d'air (52) comporte des moyens (20) de chauffage de l'alimentation d'air servant à actionner l'aspirateur (18).

5. Dispositif selon la revendication 3 ou la revendication 4, dans lequel le conduit d'alimentation d'air (52) est agencé pour diriger aussi l'alimentation d'air vers l'analyseur de concentration en combustibles (14) pour diluer le courant d'échantillon s'écoulant vers l'analyseur de concentration en combustibles.

6. Dispositif selon la revendication 5, comportant des moyens (22) de chauffage du courant dilué d'échantillon s'écoulant vers l'analyseur de concentration en combustibles (14).

7. Dispositif selon la revendication 5 ou la revendication 6, comportant un orifice à air supplémentaire (32) situé en aval de l'orifice à air (30) cité en premier lieu et dimensionné de façon à faire arriver environ 200 cm$^3$/mn d'air de dilution à l'analyseur de concentration en combustibles (14).

8. Dispositif selon l'une quelconque des revendications précédentes, dans lequel les moyens de répartition proportionnelle comprennent une série d'orifices (24, 26, 28) placés à l'intérieur de circuits (36, 38, 40) des moyens de canalisation pour répartir proportionnellement le courant d'échantillon entre l'analyseur de concentration en oxygène (16) et l'analyseur de concentration en combustibles (14).

9. Dispositif selon l'une quelconque des revendications précédentes, dans lequel les moyens de répartition proportionnelle assurent la répartition proportionnelle du courant d'échantillon entre l'analyseur de concentration en oxygène (16) et l'analyseur de concentration en combusibles (14) selon un rapport d'environ 3 à 2.

10. Dispositif selon la revendication 9, dans lequel le débit d'échantillon est d'environ 1200 cm$^3$/mn vers l'analyseur de concentration en oxygéne (16) et d'environ 800 cm$^3$/mn vers l'analyseur de concentration en combustibles (14).

0 096 557